**Europäisches Patentamt**

**European Patent Office**

**Office européen des brevets**

(19)

(11) Veröffentlichungsnummer: **0 396 883**
**A2**

(12) ## EUROPÄISCHE PATENTANMELDUNG

(21) Anmeldenummer: **90105305.8**

(22) Anmeldetag: **21.03.90**

(51) Int. Cl.⁵: **A61F 2/30**

(30) Priorität: **11.05.89 DE 8905922 U**

(43) Veröffentlichungstag der Anmeldung:
**14.11.90 Patentblatt 90/46**

(84) Benannte Vertragsstaaten:
**CH DE FR GB IT LI**

(71) Anmelder: **Vécsei, Vilmos, Prof. Dr.**
**Höfergasse 18/A**
**A-1090 Wien(AT)**

Anmelder: **Obersteiner, Karl**
**Zollstrasse 3**
**CH-9434 Au(CH)**

(72) Erfinder: **Vécsei, Vilmos, Prof. Dr.**
**Höfergasse 18/A**
**A-1090 Wien(AT)**
Erfinder: **Obersteiner, Karl**
**Zollstrasse 3**
**CH-9434 Au(CH)**

(74) Vertreter: **Dipl.-Ing. H. Hauck Dipl.-Phys. W.**
**Schmitz Dipl.-Ing. E. Graalfs Dipl.-Ing. W.**
**Wehnert Dr.-Ing. W. Döring**
**Neuer Wall 41**
**D-2000 Hamburg 36(DE)**

(54) **Gelenkprothese.**

(57) Gelenkprothese, bei der zum Verankern in der Knochensubstanz Zapfen an jedem Gelenkteil angeordnet sind, dadurch gekennzeichnet, daß die Zapfen (3) mit am Umfang angeordneten Längsstäben und mit Querstäben zwischen den Längsstäben fachwerkartig ausgebildet sind.

FIG.1

EP 0 396 883 A2

# Gelenkprothese

Die Erfindung betrifft eine Gelenkprothese nach dem Oberbegriff des Anspruchs 1.

Bei den Gelenkprothesen gemäß der Erfindung handelt es sich insbesondere um eine Knieprothese, doch können auch Sprunggelenkprothesen oder Ellenbogenprothesen mit der Anordnung gemäß der Erfindung versehen werden. In jedem Fall müssen die beiden Gelenkteile sicher im Knochen verankert werden. Hierzu sind am Gelenkteil Zapfen befestigt, die in Bohrungen des Knochens eingesetzt werden und mit dem Knochen verwachsen. Die Zapfen können mit Oberflächenunebenheiten, Löchern oder dergleichen versehen sein, damit die Verwachsung mit dem Knochen verankerungssicher erfolgt.

Der Erfindung liegt die Aufgabe zugrunde, die Gelenkteile so auszubilden, daß eine verankerungssichere Befestigung erfolgen kann.

Diese Aufgabe wird durch die Merkmale des Anspruchs 1 gelöst. Ein besonderer Vorteil der Erfindung liegt darin, daß der fachwerkartig aufgebaute Zapfen oder Schaft des Gelenkteils zur Aufnahme von Spongiosa strukturiert und damit besonders geeignet ist. Die Prothese wird somit zementlos implantiert und die Hohlräume innerhalb des Fachwerks werden wärend der Operation mit Spongiosa gefüllt. Hierdurch wird das Einwachsen der Prothese in die Knochensubstanz begünstigt.

Vorteilhafte Ausgestaltungen der Erfindungsind in den Unteransprüchen gekennzeichnet.

So ist es besonders vorteilhaft, auch die innere für den Knochenkontakt bestimmte, also der Gelenkfläche abgekehrte Seite des Gelenkteils fachwerkartig auszubilden, um auch in diesem Bereiche Aufnahmeräume für Spongiosa zu bilden und ein verankerungssicheres Einwachsen zu ermöglichen.

Das aus den Quer- und Längsstäben bestehende Fachwerk ist ausreichend steif, und Anzahl bzw. Lage der Stäbe sind so gewählt, daß das Festigkeits- bzw. Elastizitätsverhalten des natürlichen Knochens nachgebildet wird.

Die fachwerkartigen Zapfen lassen sich durch Gießen oder Sintern herstellen. Vorzugsweise werden die Zapfen stirnseitig an dem zugehörigen Gelenkteil, beispielsweise durch Schraubenverbindungen, befestigt. Ferner lassen sich die Fachwerke mit einem Überzug aus einem inerten Material versehen, beispielsweise aus Hydroxylapatit, um das Einwachsen in die Knochensubstanz zu verbessern.

Ausführungsbeispiele sind nachstehend anhand der Zeichnung näher erläutert. Es zeigt

Fig. 1 die Seitenansicht des femurseitigen Gelenkteils einer Knieprothese,

Fig. 2 eine perspektivische Ansicht des Gelenkteils gemäß Fig. 1,

Fig. 3 eine Vorderansicht des tibiaseitigen Gelenkteils der Knieprothese,

Fig. 4 eine Seitenansicht eines Fachwerks für einen Zapfen in vergrößertem Maßstab,

Fig. 5 eine Draufsicht und Seitenansicht eines Fachwerks auf einem Gelenkteil, und

Fig. 6 eine Ansicht der Patellarkomponente.

Es sei vorausgeschickt, daß jedes der beschriebenen Merkmale für sich oder in Verbindung mit Merkmalen der Ansprüche von erfindungswesentlicher Bedeutung ist.

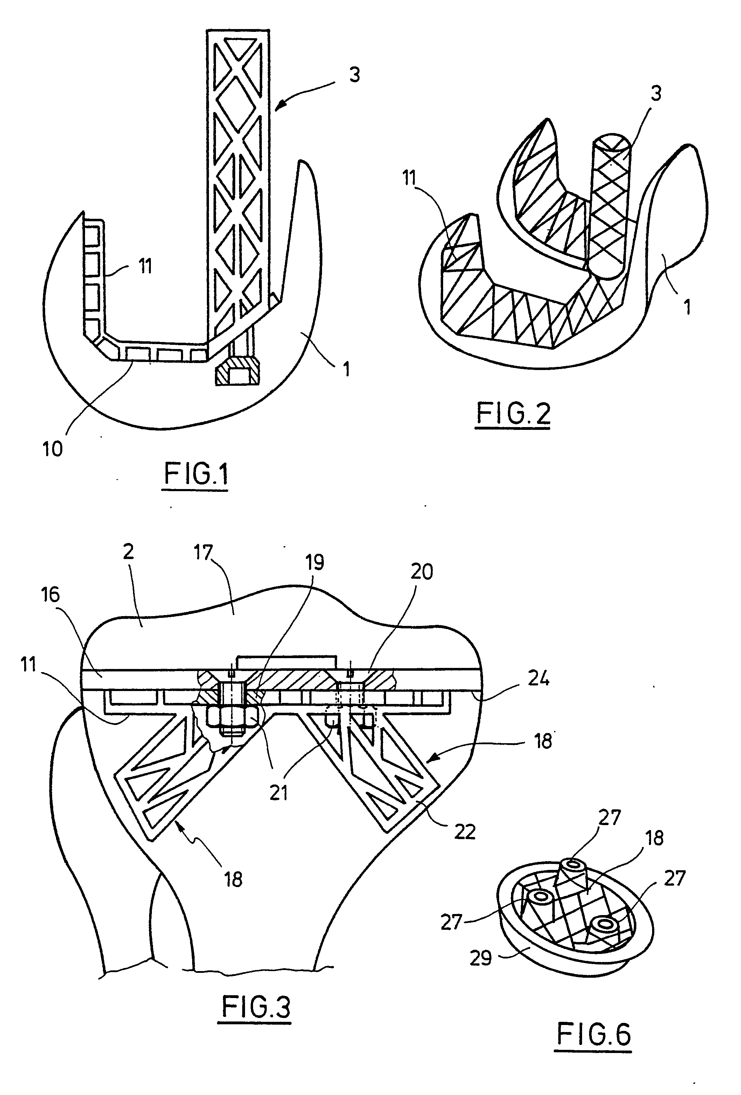

In den Figuren 1 und 2 ist ein am Oberschenkel zu befestigendes Gelenkteil 1 einer Knieprothese dargestellt. Das Gelenkteil selbst weist die bekannte Form auf und ist dazu bestimmt, mit dem in Fig. 3 dargestellten am Unterschenkel zu befestigenden Gelenkteil 2 drehbar und gleitend zusammenzuwirken. Zum Befestigen des Gelenkteils 1 am Oberschenkelknochen ist ein Zapfen 3 vorgesehen, der in einem im Oberschenkelknochen gebohrten Kanal einsitzt.

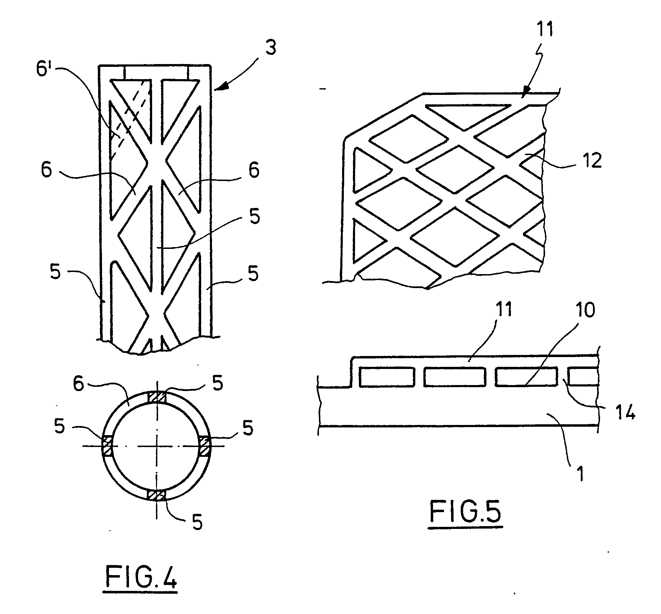

Der Zapfen 3 ist als Hohlzapfen ausgebildet und besteht aus einem Fachwerk, das in Fig. 4 im vergrößerten Maßstab dargestellt ist. Hiernach besteht der Zapfen 3 aus vier am Umfang gleichmäßig verteilten Längsstäben 5, die über Querstäbe 6 miteinander verbunden sind. Die Querstäbe 6 verlaufen schräg und kreuzen sich im Bereich der Längsstäbe 5, wie dies in Fig. 4 dargestellt ist. Das Fachwerk von Quer- und Längsstäben läßt sich auch so ausführen, wie es in der Seitenansicht des Zapfens 3 oben links dargestellt ist,wonach weitere Querstäbe 6′ zwischen den Längsstäben 5 eingefügt werden, wie dies gestrichelt angedeutet ist, um ein engeres Fachwerk zu erhalten. Es können auch im rechten Winkel zu den Längsstäben verlaufende Querstäbe vorgesehen sein. Letztlich kommt es darauf an, daß das Fachwerk dem Zapfen eine ausreichende Festigkeit verleiht und andererseits durch das Fachwerk Hohl- und Zwischenräume gebildet werden, die zur Aufnahme von Spongiosa strukturiert sind.

Ebenso ist die innere, zum Knochenkontakt bestimmte, der Gelenkfläche abgekehrte Seite 10 des Gelenkteils 1 mit einem Fachwerknetz versehen, wie dies im einzelnen in Fig. 5 dargestellt ist. Das Fachwerk 11 besteht ebenfalls aus gitterartig angeordneten, sich kreuzenden Stäben 12, die über kurze Stege 14 an der Gelenkseite 10 befestigt sind. Auf diese Weise entstehen wiederum Hohlräume zwischen dem Fachwerk 11 und dem Gelenkteil 1,die mit Spongiosa gefüllt werden und das Einwachsen der Prothese in die Knochensubstanz begünstigen. In Fig. 3 besteht der am Schienbein zu befestigende Gelenkteil der Knieprothese aus einer Metallplatte 16, auf der die aus Kunststoff bestehende Gleitfläche 17 befestigt ist. An der Metallplatte 16 sind zwei schräg verlaufende Zapfen 18 befestigt. Die Zapfen 18 sind ebenfalls als Hohlzapfen in Fachwerkbauart ausgeführt. Die der Platte 16 zugekehrte Stirnseite der Zapfen verläuft schräg und ist mit einer Scheibe 19 abgeschlossen. Mit einer Schraube 20 und einer innerhalb des Zapfens angeordneten Mutter 21 wird der Zapfen mit seiner schrägen Stirnfläche 19 an der Platte 16 befestigt. Am anderen Stirnende ist jeder Zapfen 18 ebenfalls mit einer Scheibe 22 versehen, die zur Aussteifung des Fachwerks dient und gegenüber der Längsachse des Zapfens unter einem Winkel geneigt sein kann, um eine zusätzliche Abstützung des Gelenkteils am Ende der Bohrung im Knochen zu erzielen. Auch die Zapfen 18 sind aus Längs- und Querstäben aufgebaut.

Die dem Knochen zugekehrte Seite 24 der Trägerplatte 16 ist ebenfalls mit einem der Fig.5 entsprechenden erhabenen Fachwerk 11 versehen.

In der in Fig. 3 dargestellten entsprechenden Weise ist auch der Zapfen 3 am Gelenkteil 1 befestigt. Die Fachwerkstrukturen werden vorzugsweise im Sinter- oder Gießverfahren hergestellt und sind mit einer Oberflächenbeschichtung versehen, die das Einwachsen in die Knochensubstanz fördert. Ergänzend ist in Fig. 6 noch die Patellarkomponente 26 der Knieprothese dargestellt, wobei die der Knochensubstanz zugekehrte Seite mit zwei oder drei fachwerkartig strukturierten Hohlzapfen 27 und mit einem entsprechenden Fachwerk 28 versehen ist, während die Gegenseite mit einem körperverträglichen Kunststoff als Gleitkomponente zur femoralen Gelenkbildung bestückt ist.

## Ansprüche

1. Gelenkprothese, bei der zum Verankern in der Knochensubstanz Zapfen an jedem Gelenkteil angeordnet sind, dadurch gekennzeichnet, daß die Zapfen (3, 18, 27) mit am Umfang angeordneten Längsstäben und mit Querstäben zwischen den Längsstäben fachwerkartig ausgebildet sind.

2. Gelenkprothese nach Anspruch 1, dadurch gekennzeichnet, daß die Querstäbe (6) jeweils benachbarte Längsstäbe (5) verbinden.

3. Gelenkprothese nach Anspruch 1 oder 2, dadurch gekennzeichnet, daß die Querstäbe (6) sich schräg unter einem Winkel kreuzend angeordnet sind.

4. Gelenkprothese nach Anspruch 3, dadurch gekennzeichnet, daß sich die Querstäbe (6) im Bereich der Längsstäbe (5) kreuzen.

5. Gelenkprothese nach einem der Ansprüche 1 bis 4, dadurch gekennzeichnet, daß die Enden der Zapfen scheibenförmig ausgebildet sind.

6. Gelenkprothese nach einem der Ansprüche 1 bis 5, dadurch gekennzeichnet, daß an der den Gelenkflächen abgekehrten Seite (10, 24) der Gelenkteile ein aus sich kreuzenden Stäben bestehendes Fachwerk (11, 28) angeordnet ist.

7. Gelenkprothese nach Anspruch 6, dadurch gekennzeichnet, daß das Fachwerk (11, 28) an den Kreuzungsstellen der Stäbe über kurze Stege (14) mit dem Gelenkteil verbunden ist.

8. Gelenkprothese nach einem der Ansprüche 1 bis 7, dadurch gekennzeichnet, daß die Fachwerkstrukturen als Gußteil oder Sinterteil aus einer körperverträglichen Metallegierung oder einem entsprechenden Metall geformt sind.

9. Gelenkprothese nach einem der Ansprüche 1 bis 8, dadurch gekennzeichnet, daß die Fachwerkstrukturen und der Knochensubstanz zugekehrten Flächen der Gelenkteile mit einem Überzug aus Hydroxylapatit oder einem ähnlichen inerten Material versehen sind.

FIG.1

FIG.2

FIG.3

FIG.6

FIG.4

FIG.5